# FASCICULE DE BREVET EUROPEEN

(11) **EP 1 385 512 B1**
(45) Date de publication et mention de la délivrance du brevet: **28.12.2005**
(21) Numéro de dépôt: 02732841.8
(22) Date de dépôt: 26.04.2002
(51) Int. Cl.: A61K 31/437, A61P 35/00, A61K 31/519

(54) **UTILISATION DE DERIVES DE PYRIDOINDOLONE POUR LA PREPARATION DE MEDICAMENTS ANTICANCEREUX**
VERWENDUNG VON PYRIDOINDOLONDERIVATEN ZUR HERSTELLUNG VON ANTIKREBSMITTELN
USE OF PYRIDOINDOLONE DERIVATIVES FOR PREPARING ANTICANCER MEDICINES

(30) Priorité: 27.04.2001 FR 0105843
(43) Date de publication de la demande: 04.02.2004
(73) Titulaire: Sanofi-Aventis, 75013 Paris (FR)
(72) Inventeur: BOURRIE, Bernard, F-34980 Saint Gely du Fesc (FR); CASELLAS, Pierre, F-34090 Montpellier (FR); DEROCQ, Jean-Marie, F-34570 Murviel les Montpellier (FR)
(74) Mandataire: Varady, Peter
(86) Numéro de dépôt international: PCT/FR2002/001449
(87) Numéro de publication internationale: WO 2002/087574

(56) Documents cités:
- WO-A-99/51597
- FR-A- 2 003 999
- FR-A- 2 765 581
- US-A- 4 835 160
- US-A- 5 880 126
- DATABASE WPI Week 198213 Derwent Publications Ltd., London, GB; AN 1982-25808e XP002184731 & SU 833 971 A (LE KHIM FARMA I) 30 mai 1981 (1981-05-30)

## Description

La présente invention a pour objet une nouvelle application thérapeutique de dérivés de pyridoindolone.

Le document FR 97 08409 décrit des composés de formule : 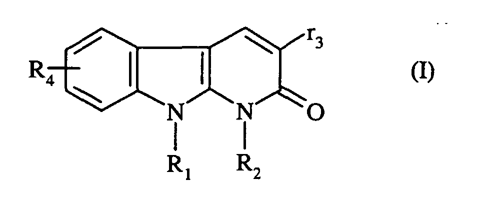 dans laquelle :
- R₁ représente un atome d'hydrogène ou un groupe méthyle ou éthyle ;
- R₂ représente un groupe méthyle ou éthyle ; ou bien
- R₁ et R₂ forment ensemble un groupe (CH₂)₃ ;
- r₃ représente, soit un groupe phényle éventuellement substitué par un atome d'halogène ou un groupe méthyle ou méthoxy, soit un groupe thiényle ;
- R₄ représente un atome d'hydrogène ou de chlore ou un groupe méthyle ou méthoxy.

Dans la description de ce document, il est mentionné que les composés de formule (I) ayant une affinité pour les sites modulateurs oméga associés aux récepteurs GABA_{A}, peuvent être utilisés dans le traitement d'affections liées aux désordres de la transmission gabaergique associés aux sous-types de récepteurs GABA_{A}, tels que l'anxiété, les troubles du sommeil, l'épilepsie etc...

Il a maintenant été trouvé que les composés de formule (I) sont des agents anticancéreux, inhibant la prolifération des cellules tumorales, qui ont des propriétés antimitotiques.

L'invention a pour objet l'utilisation des composés de formule (I) tels que définis ci-dessus, de leurs sels pharmaceutiquement acceptables, hydrates ou solvats, pour la préparation de médicaments utiles comme agents anticancéreux.

Des composés préférés selon l'invention sont les composés de formule : 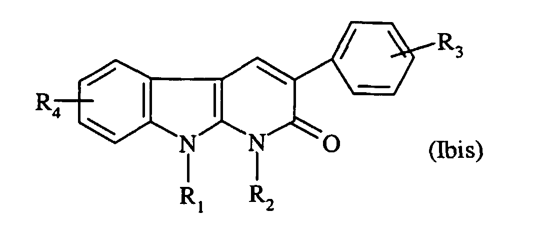 dans laquelle :
- R₁ représente un atome d'hydrogène, un groupe méthyle ou éthyle ;
- R₂ représente un groupe méthyle ou éthyle ; ou bien
- R₁ et R₂ forment ensemble un groupe (CH₂)₃ ;
- R₃ représente un atome d'hydrogène ou d'halogène ou un groupe méthyle ou méthoxy ;
- R₄ représente un atome d'hydrogène ou de chlore ou un groupe méthyle ou méthoxy.

Des composés particulièrement préférés selon l'invention sont les composés de formule: 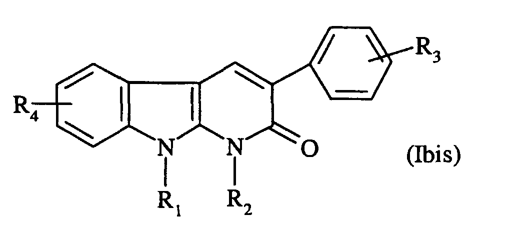 dans laquelle :
- R₁ représente un atome d'hydrogène, un groupe méthyle ou éthyle ;
- R₂ représente un groupe méthyle ou éthyle ;
- R₃ représente un atome d'hydrogène ou d'halogène ou un groupe méthyle ou méthoxy ;
- R₄ représente un atome d'hydrogène ou de chlore ou un groupe méthyle ou méthoxy.

Des composés plus particulièrement préférés selon l'invention sont les composés de formule: 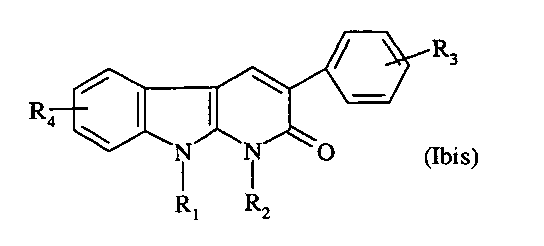 dans laquelle :
- R₁ représente un groupe méthyle ou éthyle ;
- R₂ représente un groupe méthyle ou éthyle ;
- R₃ représente un atome d'hydrogène ou d'halogène ou un groupe méthyle ou méthoxy ;
- R₄ représente un atome d'hydrogène ou de chlore ou un groupe méthyle ou méthoxy.
A titre d'exemple, des composés de l'invention sont les suivants :
- la 6-chloro-1,9-diméthyl-3-phényl-1,9-dihydro-2H-pyrido[2,3-b]indol-2-one ;
   F = 178,5-179-5°C ;
- la 3-(4-methoxyphényl)-1,9-diméthyl-1,9-dihydro-2H-pyrido[2,3-b]indol-2-one ;
   F = 166-167°C ;
- la 1,6,9-triméthyl-3-(3-thiényl)-1,9-dihydro-2H-pyrido[2,3-b]indol-2-one ;
   RMN (200 MHz) : 2,6 ppm : s : 3H ; 4,1 ppm : s : 3H ; 4,2 ppm : s : 3H ; 7,1 ppm : d : 1H ; 7,4-7,9 ppm : m : 4H ; 8,3 ppm : d : 1H ; 8,7 ppm : s : 1H.
- la 1,6,9-triméthyl-3-phényl-1,9-dihydro-2H-pyrido[2,3-b]indol-2-one ;
   F = 198-199°C ;
- la 1,6-diméthyl-3-phényl-1,9-dihydro-2H-pyrido[2,3-b]indol-2-one ;
   RMN (200 MH₂) : 2,5 ppm : s : 3H ; 3,8 ppm : s : 3H ; 7,1 ppm : d : 1H ; 7,3-7,5 ppm : m : 4H ; 7,75 ppm : d : 2H ; 7,8 ppm : s : 1H ; 8,4 ppm : s : 1H ; 11,8 ppm : s : 1H.

Les composés de formule (I) sont préparés selon le procédé décrit dans le document FR 97 08409.

Les composés de formule (I) selon la présente invention ont été testés *in vitro* sur une lignée cellulaire humaine de cancer de sein : la lignée MDA-MB-231 disponible auprès de l'American Type Culture Collection (référence HTB26).

L'évaluation de l'effet antiprolifératif est effectuée selon J.M. Derocq et al., FEBS Letters, 1998, 425, 419-425 : on mesure le taux d'incorporation de la [3H]thymidine dans l'ADN des cellules traitées, après 96 heures d'incubation d'un composé de formule (I). La concentration inhibitrice 50 (CI₅₀) est définie comme la concentration qui inhibe la prolifération cellulaire de 50 %.

Les composés selon l'invention présentent une CI₅₀ généralement inférieure à 10 µM sur la lignée MDA-MB-231.

Les composés de formule (I) ont également été testés sur une autre lignée cellulaire humaine de cancer du sein, dite lignée multi-résistante MDR, (de l'anglais multi-drug-resistant) et appelée MDA-A₁. Cette lignée est décrite par E. Collomb, C. Dussert et P.M. Martin dans Cytometry, 1991, 12(1), 15-25.

Le terme "multi-résistant" qui qualifie cette lignée, signifie que ladite lignée est peu sensible d'une manière générale aux drogues de chimiothérapie communément utilisées et en particulier aux antimitotiques d'origine naturelle tels que le paclitaxel, la vincristine, la vinblastine.

Les composés selon l'invention présentent une CI₅₀ généralement inférieure à 10 µM sur la lignée multi-résistante MDA-A₁.

Ainsi, selon la présente invention, les composés de formule (I) inhibent la prolifération des cellules tumorales y compris la prolifération des cellules présentant une multi-résistance.

Plusieurs composés selon l'invention ont été évalués *in vivo* sur un modèle de xénogreffe de tumeurs humaines implantées en sous-cutané sur la souris immuno-déprimée SCID (de l'anglais : Severe Combined Immuno Deficiency).

Le traitement des animaux avec un composé selon l'invention débute 6 à 7 jours après l'implantation, lorsque la tumeur atteint une masse tumorale d'environ 60 mg. Le composé, en solution dans un solvant, est alors administré par voie orale.

L'activité antitumorale est évaluée lorsque la masse tumorale moyenne atteint environ 1000 mg chez les animaux contrôles, traités avec le solvant uniquement : on mesure le rapport T/C, T représentant le poids moyen des tumeurs chez les animaux traités et C représentant le poids moyen des tumeurs chez les animaux contrôles. Un rapport T/C inférieur ou égal à 42 % est considéré comme indicateur d'une activité antitumorale significative selon Stuart T et al., dans J. Med. Chem., 2001, 44 (11), 1758-1776. Pour une dose journalière cumulée, administrée, comprise entre 50 et 300 mg/kg, certains composés selon l'invention ont conduit à un rapport T/C inférieur à 20%.

Les composés de formule (I), leurs sels pharmaceutiquement acceptables, hydrates ou solvats, sont utiles pour prévenir ou traiter les maladies causées ou exacerbées par la prolifération des cellules tumorales, telles que les tumeurs primaires ou métastasiques, des carcinomes et cancers, en particulier : cancer du sein ; cancer du poumon ; cancer de l'intestin grêle, cancer du colon et du rectum ; cancer des voies respiratoires, de l'oropharynx et de l'hypopharynx ; cancer de l'oesophage ; cancer du foie, cancer de l'estomac, cancer des canaux biliaires, cancer de la vésicule biliaire, cancer du pancréas ; cancers des voies urinaires y compris rein, urothelium, et vessie ; cancers du tractus génital féminin y compris cancer de l'utérus, du col de l'utérus, des ovaires, chloriocarcinome et trophoblastome ; cancers du tractus génital masculin y compris cancer de la prostate, des vésicules séminales, des testicules, tumeurs des cellules germinales ; cancers des glandes endocrines y compris cancer de la thyroïde, de l'hypophyse, des glandes surrénales ; cancers de la peau y compris hémangiomes, mélanomes, sarcomes, incluant le sarcome de Kaposi ; tumeurs du cerveau, des nerfs, des yeux, des méninges, incluant astrocytomes, gliomes, glioblastomes, rétinoblastomes, neurinomes, neuroblastomes, schwannomes, méningiomes ; tumeurs venant de tumeurs malignes hématopoïétiques incluant leucémies, chloromes, plasmacytomes, mycosis fongoïde, lymphome ou leucémie des cellules T, lymphome non hodgkinien, hémopathies malignes, myélomes.

Les composés de formule (I) ci-dessus peuvent être utilisés à des doses journalières de 0,002 à 2000 mg par kilogramme de poids corporel du mammifère à traiter, de préférence à des doses journalières de 0,1 à 300 mg/kg. Chez l'être humain, la dose peut varier de préférence de 0,02 à 10000 mg par jour, plus particulièrement de 1 à 3000 mg, selon l'âge du sujet à traiter ou le type de traitement (prophylactique ou curatif).

Selon un autre de ses aspects, la présente invention concerne des compositions pharmaceutiques comprenant, en tant que principe actif, une dose efficace d'au moins un composé selon l'invention, ou un sel pharmaceutiquement acceptable, un hydrate ou un solvat dudit composé, ainsi qu'un ou plusieurs excipients pharmaceutiquement acceptables.
Lesdits excipients sont choisis selon la forme pharmaceutique et le mode d'administration souhaité, parmi les excipients habituels qui sont connus dans l'art antérieur.
Les compositions pharmaceutiques de la présente invention peuvent être préparées pour l'administration orale, sublinguale, sous-cutanée, intramusculaire, intraveineuse, topique, locale, intratrachéale, intranasale, transdermique ou rectale, aux animaux et aux êtres humains pour la prévention ou le traitement des maladies ci-dessus.
Les formes d'administration appropriées comprennent les formes orales telles que les comprimés, les gélules molles ou dures, les poudres, les granules et les solutions ou suspensions orales, les formes pour administration sublinguale, buccale, intratrachéale, intraoculaire, intranasale, par inhalation, les formes pour administration topique, transdermique, sous-cutanée, intramusculaire ou intraveineuse, les formes pour administration rectale et les implants. Pour l'application topique, on peut utiliser les composés selon l'invention dans des crèmes, gels, pommades ou lotions.

Selon la pratique habituelle, le dosage approprié à chaque patient est déterminé par le médecin selon le mode d'administration, l'âge, le poids et la réponse dudit patient.

## Revendications

1. Utilisation d'un composé de formule : 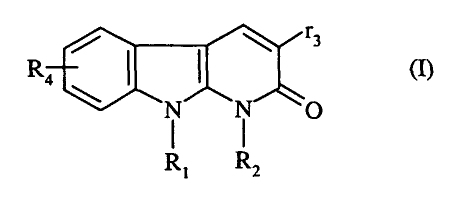 dans laquelle :
- R₁ représente un atome d'hydrogène ou un groupe méthyle ou éthyle ;
- R₂ représente un groupe méthyle ou éthyle ; ou bien
- R₁ et R₂ forment ensemble un groupe (CH₂)₃ ;
- r₃ représente, soit un groupe phényle éventuellement substitué par un atome d'halogène ou un groupe méthyle ou méthoxy, soit un groupe thiényle ;
- R₄ représente un atome d'hydrogène ou de chlore ou un groupe méthyle ou méthoxy ;
pour la préparation de médicaments utiles comme agents anticancéreux.

2. Utilisation selon la revendication 1 d'un composé de formule : 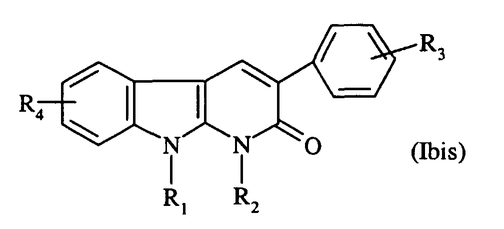 dans laquelle :
- R₁ représente un atome d'hydrogène, un groupe méthyle ou éthyle ;
- R₂ représente un groupe méthyle ou éthyle ; ou bien
- R₁ et R₂ forment ensemble un groupe (CH₂)₃ ;
- R₃ représente un atome d'hydrogène ou d'halogène ou un groupe méthyle ou méthoxy ;
- R₄ représente un atome d'hydrogène ou de chlore ou un groupe méthyle ou méthoxy.

3. Utilisation selon la revendication 1 ou la revendication 2 d'un composé de formule : 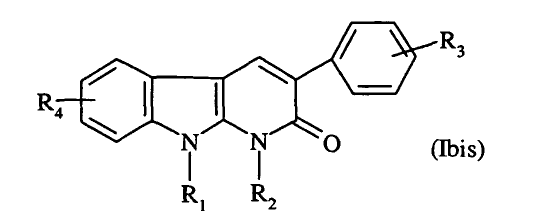 dans laquelle :
- R₁ représente un atome d'hydrogène, un groupe méthyle ou éthyle ;
- R₂ représente un groupe méthyle ou éthyle ;
- R₃ représente un atome d'hydrogène ou d'halogène ou un groupe méthyle ou méthoxy ;
- R₄ représente un atome d'hydrogène ou de chlore ou un groupe méthyle ou méthoxy.

4. Utilisation de l'un des composés cités ci-après :
- la 6-chloro-1,9-diméthyl-3-phényl-1,9-dihydro-2H-pyrido[2,3-b]indol-2-one ;
- la 3-(4-methoxyphényl)-1,9-diméthyl-1,9-dihydro-2H-pyrido[2,3-b]indol-2-one ;
- la 1,6,9-triméthyl-3-(3-thiényl)-1,9-dihydro-2H-pyrido[2,3-b]indol-2-one ;
- la 1,6,9-triméthyl-3-phényl-1,9-dihydro-2H-pyrido[2,3-b]indol-2-one ;
- la 1,6-diméthyl-3-phényl-1,9-dihydro-2H-pyrido[2,3-b]indol-2-one ;
pour la préparation de médicaments utiles comme agents anticancéreux.

## Patentansprüche

1. Verwendung einer Verbindung der Formel: 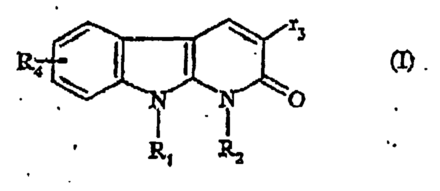 worin:
- R₁ ein Wasserstoffatom oder eine Methyl- oder Ethylgruppe darstellt;
- R₂ eine Methyl- oder Ethylgruppe darstellt; oder aber
- R₁ und R₂ zusammen eine (CH₂)₃-Gruppe bilden;
- r₃ entweder eine gegebenenfalls mit einem Halogenatom oder einer Methyl- oder Methoxygruppe substituierte Phenylgruppe oder eine Thienylgruppe darstellt;
- R₄ ein Wasserstoff- oder Chloratom oder eine Methyl- oder Methoxygruppe darstellt;
zur Herstellung von Arzneimitteln, die sich als Antikrebs-Mittel eignen.

2. Verwendung gemäß Anspruch 1 einer Verbindung der Formel: 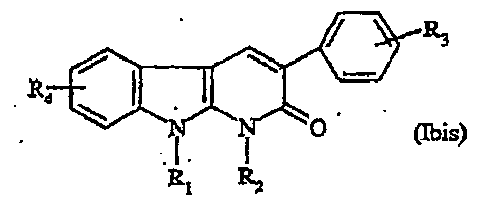 worin:
- R₁ ein Wasserstoffatom, eine Methyl- oder Ethylgruppe darstellt;
- R₂ eine Methyl- oder Ethylgruppe darstellt; oder aber
- R₁ und R₂ zusammen eine (CH₂)₃-Gruppe bilden;
- R₃ ein Wasserstoff- oder Halogenatom oder eine Methyl- oder Methoxygruppe darstellt;
- R₄ ein Wasserstoff- oder Chloratom oder eine Methyl- oder Methoxygruppe darstellt.

3. Verwendung gemäß Anspruch 1 oder Anspruch 2 einer Verbindung der Formel: 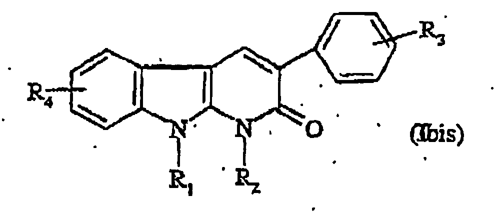 worin:
- R₁ ein Wasserstoffatom, eine Methyl- oder Ethylgruppe darstellt;
- R₂ eine Methyl- oder Ethylgruppe darstellt;
- R₃ ein Wasserstoff- oder Halogenatom oder eine Methyl- oder Methoxygruppe darstellt;
- R₄ ein Wasserstoff- oder Chloratom oder eine Methyl- oder Methoxygruppe darstellt.

4. Verwendung einer der im Folgenden angeführten Verbindungen:
- 6-Chlor-1,9-dimethyl-3-phenyl-1,9-dihydro-2H-pyrido[2,3-b]indol-2-on;
- 3-(4-Methoxyphenyl)-1,9-dimethyl-1,9-dihydro-2H-pyrido[2,3-b]indol-2-on;
- 1,6,9-Trimethyl-3-(3-thienyl)-1,9-dihydro-2H-pyrido[2,3-b]indol-2-on;
- 1,6,9-Trimethyl-3-phenyl-1,9-dihydro-2H-pyrido[2,3-b]indol-2-on;
- 1,6-Dimethyl-3-phenyl-1,9-dihydro-2H-pyrido[2,3-b]indol-2-on;
zur Herstellung von Arzneimitteln, die sich als Antikrebs-Mittel eignen.

## Claims

1. Use of a compound of formula: 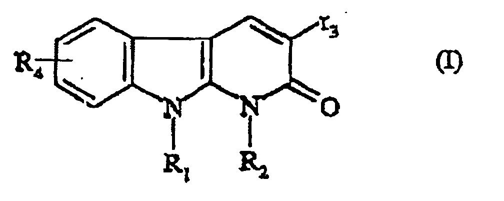 in which:
- R₁ represents a hydrogen atom or a methyl or ethyl group;
- R₂ represents a methyl or ethyl group; or
- R₁ and R₂ together form a (CH₂)₃ group;
- r₃ represents either a phenyl group optionally substituted with a halogen atom or a methyl or methoxy group, or a thienyl group;
- R₄ represents a hydrogen or chlorine atom or a methyl or methoxy group;
for the preparation of medicinal products that are useful as anticancer agents.

2. Use according to Claim 1, of a compound of formula: 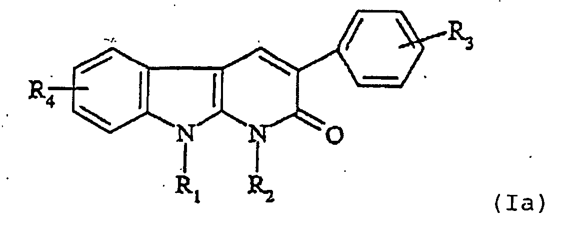 in which:
- R₁ represents a hydrogen atom or a methyl or ethyl group;
- R₂ represents a methyl or ethyl group; or
- R₁ and R₂ together form a (CH₂)₃ group;
- R₃ represents a hydrogen or halogen atom or a methyl or methoxy group;
- R₄ represents a hydrogen or chlorine atom or a methyl or methoxy group.

3. Use according to Claim 1 or Claim 2, of a compound of formula: 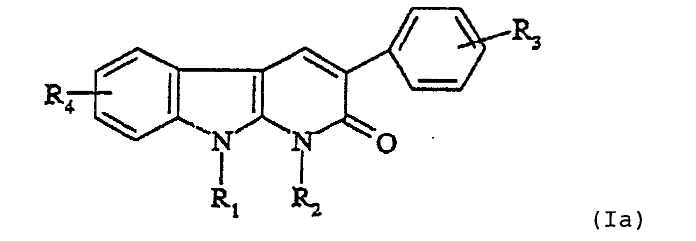 in which:
- R₁ represents a hydrogen atom or a methyl or ethyl group;
- R₂ represents a methyl or ethyl group;
- R₃ represents a hydrogen or halogen atom or a methyl or methoxy group;
- R₄ represents a hydrogen or chlorine atom or a methyl or methoxy group.

4. Use of one of the compounds mentioned below:
- 6-chloro-1,9-dimethyl-3-phenyl-1,9-dihydro-2H-pyrido[2,3-b]indol-2-one;
- 3-(4-methoxyphenyl)-1,9-dimethyl-1,9-dihydro-2H-pyrido[2,3-b]indol-2-one;
- 1,6,9-trimethyl-3-(3-thienyl)-1,9-dihydro-2H-pyrido[2,3-b]indol-2-one;
- 1,6,9-trimethyl-3-phenyl-1,9-dihydro-2H-pyrido[2,3-b]indol-2-one;
- 1,6-dimethyl-3-phenyl-1,9-dihydro-2H-pyrido[2,3-b]indol-2-one;
for the preparation of medicinal products that are useful as anticancer agents.
